# EUROPEAN PATENT SPECIFICATION

(11) **EP 2 252 687 B1**
(45) Date of publication and mention of the grant of the patent: **23.10.2013**
(21) Application number: 09718831.2
(22) Date of filing: 13.03.2009
(51) Int. Cl.: C12N 9/12, C12N 15/54, C12N 15/63, C12N 1/21, C12Q 1/68

(54) **A THERMOSTABLE DNA POLYMERASE FROM PALAEOCOCCUS HELGESONII**
THERMOSTABILE DNA POLYMERASE VON PALAEOCOCCUS HELGESONII
ADN POLYMÉRASE THERMOSTABLE DE PALAEOCOCCUS HELGESONII

(30) Priority: 14.03.2008 GB 0804721; 14.03.2008 US 69429 P
(43) Date of publication of application: 24.11.2010
(73) Proprietor: Genesys Ltd, Surrey GU16 7PL (GB)
(72) Inventor: CLARK, Duncan, Surrey GU16 7PL (GB); MORANT, Nicholas, Surrey GU16 7PL (GB)
(74) Representative: Turner, Rhiannon Rosalind
(86) International application number: PCT/GB2009/050247
(87) International publication number: WO 2009/112868

(56) References cited:
- DATABASE Geneseq [Online] 26 July 2007 (2007-07-26), "Thermococcus sp. TNA1 polymerase SEQ ID NO 2." XP002534727 retrieved from EBI accession no. GSP:AFS77634 Database accession no. AFS77634 -& WO 2007/043769 A (KOREA OCEAN RES & DEV I [KR]; LEE JUNG HYUN [KR]; KWON SUK TAE [KR]; K) 19 April 2007 (2007-04-19)
- DATABASE Geneseq [Online] 23 February 2006 (2006-02-23), "Thermococcus sp. DNA polymerase SEQ ID NO 16." XP002534728 retrieved from EBI accession no. GSP:AEE85205 Database accession no. AEE85205 -& EP 1 757 698 A (TAKARA BIO INC [JP]) 28 February 2007 (2007-02-28)
- AMEND JAN P ET AL: "Palaeococcus helgesonii sp. nov., a facultatively anaerobic, hyperthermophilic archaeon from a geothermal well on Vulcano Island, Italy." ARCHIVES OF MICROBIOLOGY, vol. 179, no. 6, June 2003 (2003-06), pages 394-401, XP002534726 ISSN: 0302-8933

## Description

### Field of Invention

The present invention relates to novel polypeptides having DNA polymerase activity, and their uses.

### Background

DNA polymerases are enzymes involved *in vivo* in DNA repair and replication, but have become an important *in vitro* diagnostic and analytical tool for the molecular biologist. The enzymes are divided into three main families, based on function and conserved amino acid sequences (see Joyce & Steitz, 1994, Ann. Rev. Biochem. 63: 777-822). In prokaryotes, the main types of DNA polymerases are DNA polymerase I, II and III. DNA polymerase I (encoded by the gene "polA" in *E. coli*) is considered to be a repair enzyme and has 5'-3' polymerase activity and often 3'-5' exonuclease proofreading activity and/or 5'-3' exonuclease activity which when present mediates nick translation during DNA repair. DNA polymerase II (encoded by the gene "polB" in *E. coli*) appears to facilitate DNA synthesis starting from a damaged template strand and thus preserves mutations. DNA polymerase III (encoded by the gene "polC" in *E. coli*) is the replication enzyme of the cell, synthesising nucleotides at a high rate (such as about 30,000 nucleotides per minute) and having no 5'-3' exonuclease activity.

Other properties of DNA polymerases are derived from their source of origin. For example, several DNA polymerases obtained from thermophilic bacteria have been found to be thermostable, retaining polymerase activity at between 45°C to 100°C, depending on the polymerase. Thermostable DNA polymerases have found wide use in methods for amplifying nucleic acid sequences by thermocycling amplification reactions such as the polymerase chain reaction (PCR) or by isothermal amplification reactions such as strand displacement amplification (SDA), nucleic acid sequence-based amplification (NASBA), self-sustained sequence replication (3SR), and loop-mediated isothermal amplification (LAMP).

The different properties of thermostable DNA polymerases, such as level of thermostability, strand displacement activity, fidelity (error rate) and binding affinity to template DNA and/or RNA and/or free nucleotides, make them suited to different types of amplification reaction. For example, thermostable (typically at temperatures up to 94°C), high-fidelity (typically with 3'-5' exonuclease proof-reading activity), processive and rapidly synthesising DNA polymerases are preferred for PCR. Enzymes which do not discriminate significantly between dideoxy and deoxy nucleotides may be preferred for sequencing. Meanwhile, isothermal amplification reactions require a DNA polymerase with strong strand displacement activity.

The proof-reading DNA polymerases currently available commercially for PCR are derived from species within either the *Pyrococcus* genus or the *Thermococcus* genus of hyperthermophilic euryarchaeota. *Archaea* are a third domain of living organisms, distinct from *Bacteria* and *Eucarya.* These organisms have been isolated predominantly from deep-sea hydrothermal vents ("black smokers") and typically have optimal growth temperatures around 85-99°C. Examples of key species from which proof-reading DNA polymerases for use in PCR have been isolated include *Thermococcus barossii*, *Thermococcus litoralis*, *Thermococcus gorgonarius, Thermococcus pacificus, Thermococcus zilligii, Thermococcus 9N7, Thermococcus fumicolans*, *Thermococcus aggregans (TY), Thermococcus peptonophilus*, *Pyrococcus furiosus*, *Pyrococcus sp.* and *Thermococcus KOD.*

Takagi et al. (Appl. Env. Microbiol. (1997) 63: 4504-4510) and EP-A-0745675 provide characterisation of the DNA polymerase found in *Pyrococcus* sp. Strain KOD1. This strain has an optimum growth temperature of 95°C. US7045328 discloses a DNA polymerase from *P. furiosus* and US5834285 a DNA polymerase from *T.litoralis.* Griffiths et al. (Prot. Exp. and Purification (2006) 52 19-30) discloses polymerases from *Thermococcus* species *T. ziglligii* and *Thermococcus* 'GT'. WO2007/043769 discloses a further polymerase from *Thermococcus* sp. NA1. Isolation and characterisation of *Paleococcus helgesonii* is described in Amend et al (2003) Arch. Microbiol. 179 394-401.

### Summary of Invention

The present invention provides in one aspect a novel thermostable DNA polymerase for use in reactions requiring DNA polymerase activity such as nucleic acid amplification reactions. The polymerase has been isolated from a new genus of hyperthermophilic euryarchaeota, the *Palaeococcus* genus, which represents a deep-branching lineage of the order Thermococcales that diverged before *Thermococcus* and *Pyrococcus.* Surprisingly, the polymerase is suitable for use in thermocycling amplification reactions, even though the optimum growth temperature for the organism is only 80°C (see below).

According to one aspect of the present invention there is provided a polypeptide having thermostable DNA polymerase activity and comprising or consisting essentially of an amino acid sequence with at least 79% identity, for example at least 80%, 85%, 90% or 95% identity, to *Palaeococcus helgesonii* DNA polymerase shown in SEQ ID NO: 1. The polypeptide may, for example, have 79%, 81%, 82%, 83%, 84%, 86%, 87%, 88%, 89% 91%, 92%, 93%, 94%, 96%, 97%, 98% or even 99% identity to SEQ ID NO: 1. Preferably, the polypeptide is isolated.

The *P. helgesonii* DNA polymerase has the following amino acid sequence:

The underlined amino acid "X" has been confirmed as being "Q" and, therefore, a preferred embodiment of the polypeptide according to the invention has the amino acid sequence:

Preferably, the polypeptide has thermostable DNA polymerase activity and comprises or consists essentially of an amino acid sequence with at least 79% identity, for example at least 80%, 85%, 90% or 95% identity, to *Palaeococcus helgesonii* DNA polymerase shown in SEQ ID NO: 39. The polypeptide may, for example, have 79%, 81%, 82%, 83%, 84%, 86%, 87%, 88%, 89% 91%, 92%, 93%, 94%, 96%, 97%, 98% or even 99% identity to SEQ ID NO: 39. Preferably, the polypeptide is isolated.

The predicted molecular weight of this 773 amino acid residue *P. helgesonii* DNA polymerase shown in SEQ ID NO: 39 is about 89,750 Daltons.

The above percentage sequence identity may be determined using the BLASTP computer program with SEQ ID NO:1 or 39 as the base sequence. This means that SEQ ID NO:1 or 39 is the sequence against which the percentage identity is determined. The BLAST software is publicly available at http://blast.ncbi.nlm.nih.gov/Blast.cgi (accessible on 12 March 2009). For example, the polypeptide may comprise or consist essentially of any contiguous 603 amino acid sequence included within SEQ ID NO:39. For example, the polypeptide may comprise from about 580 to 773, about 600 to 750 or about 650 to 700 contiguous amino acids included within SEQ ID NO:39.

The polypeptide may comprise or consist essentially of the amino acid sequence SEQ ID NO:39, or of the amino acid sequence of SEQ ID NO:39 with 1, 2, 3, 4, 5, 6, 7, 8, 9, 10, 11, 12, 13, 14, 15, or about 20 amino acids or contiguous amino acids added to or removed from any part of the polypeptide and/or 1, 2, 3, 4, 5, 6, 7, 8, 9, 10, 11, 12, 13, 14, 15 or about 20 amino acids or contiguous amino acids added to or removed from the N-terminus region and/or the C-terminus region.

*Palaeococcus helgesonii* is a facultatively anaerobic hyperthermophilic archaeon isolated from a shallow geothermal well in the southern Tyrrhenian Sea, Italy, and has a reported temperature range for growth of 45-85°C and an optimum growth temperature of about 80°C (see Amend et al., 2003, Arch. Microbiol. 179: 394-401). This organism was reported to be the second member of the *Palaeococcus* genus of hyperthermophilic euryarchaeota, and to date there are no known published reports of the identification and characterisation of a DNA polymerase from this genus. Genomic DNA (gDNA) from *P. helgesonii* has been isolated by the inventors who used a sophisticated gene walking technique to clone a DNA polymerase, considered to be a DNA polymerase II encoded by a DNA polymerase II (polB) gene.

DNA polymerase II enzymes comprise certain conserved motifs, for example, as described in Kim et al., (2007) J. Microbiol. Biotechnol. 17 1090-1097. Therefore, in a preferred embodiment, the peptide according to the invention comprises one or more of the amino acid sequences:
EX₁X₂X₁₈IKX₃FLX₁₉X₄X₂₀X₁EKDPDX₄X₅X₄TY (SEQ ID NO:28)
GX₆VKEPEX₁GLWX₂X₂₁X₅X₂₂X₈LDX₆X₁X₉LYPSIIX₄THNVSPDT (SEQ ID NO:29)
GFIPSX₅LX₁₀X₁₁L X₅X₂X₂₃RQX₁₂X₄KX₁₃KMK (SEQ ID NO:30)
DYRQX₁AX₅KX₅LANSX₆YGYX₂₄GYX₁₄X₁ (SEQ ID NO:31)
DTDGX₁₅X₁₆A (SEQ ID NO:32)
DEEGX₂₅X₄X₁₇TRGLEX₄VRRDWSX₂IAK (SEQ ID NO:33)
where:

| | |
|---|---|
| X₁=K or R | X₁₄=A or P |
| X₂=E or D | X₁₅=F or L |
| X₃=R or A | X₁₆=Y, F or H |
| X₄=V or I | X₁₇=V, T or I |
| X₅=L or I | X₁₈=M or A |
| X₆=Y or F | X₁₉=K, R or H |
| X₇=N or G | X₂₀=V, I or L |
| X₈=Y or S | X₂₁=N, G or S |
| X₉=S or A | X₂₂=V or A |
| X₁₀=G, K or E | X₂₃=E or T |
| X₁₁=N, D, H or E | X₂₄=Y or T |
| X₁₂=K or E | X₂₅=G or H |
| X₁₃=R, K or T | |

For example, the polypeptide may comprise any two, any three, any four or any five amino acid sequences selected from SEQ ID NOs: 28-33 or may comprise all of amino acid sequences SEQ ID NOs: 28-33. In a preferred embodiment, the peptide according to the invention may comprise one or both of the amino acid sequences:
LYPSIIX₄THNVSPDT (SEQ NO:34)
TRGLEX₄VRRDWSX₂IAK (SEQ ID NO:35).

The polypeptide may be suitable for carrying out a thermocycling amplification reaction, such as a polymerase chain reaction (PCR). This characteristic requires sufficient thermostability to withstand the denaturation cycle, normally 95°C.

The polypeptide of the invention may be sufficiently stable to allow it to be functional in a thermocycling reaction such as PCR (for example, as exemplified in Example 8 below). Even though *P. helgesonii* has a reported growth range of up to 88°C (see above), the inventors have surprisingly found that even a crude extract of the DNA polymerase II of SEQ ID NO:39 is sufficiently stable for use in PCR.

The polypeptide may have 3' - 5' exonuclease proofreading activity.

In some embodiments, the polypeptide may lack 5'-3' exonuclease activity.

The polypeptide of the invention may be an isolated thermostable DNA polymerase obtainable from *Palaeococcus helgesonii* and having a molecular weight of about 90,000 Daltons, or about 89,000 - about 91,000 Daltons, or an enzymatically active fragment thereof. The term "enzymatically active fragment" means a fragment of such a polymerase obtainable from *P. helgesonii* and having enzyme activity which is at least 60%, preferably at least 70%, more preferably at least 80%, yet more preferably 90%, 95%, 96%, 97%, 98%, 99% or 100% that of the full length polymerase being compared to. The given activity may be determined by any standard measure, for example, the number of bases of nucleotides of the template sequence which can be replicated in a given time period. The skilled person is routinely able to determine such properties and activities.

The polypeptide of the invention may be suitable for use in one or more reactions requiring DNA polymerase activity, for example one or more of the group consisting of: nick translation, second-strand cDNA synthesis in cDNA cloning, DNA sequencing, and thermocycling amplification reactions such as PCR.

In a further aspect of the invention the polypeptide exhibits high fidelity polymerase activity during a thermocycling amplification reaction (such as PCR). High fidelity may be defined as a PCR error rate of less than 1 nucleotide per 300 x 10⁶ amplified nucleotides, for example less than 1 nucleotide per 250 x 10⁶, 200 x 10⁶, 150 x 10⁶, 100 x 10⁶ or 50 x 10⁶ amplified nucleotides. Alternatively, the error rate of the polypeptides may be in the range 1-300 nucleotides per 10⁶ amplified nucleotides, for example 1-200, 1-100, 100-300, 200-300, 100-200 or 75-200 nucleotides per 10⁶ amplified nucleotides. Error rate may be determined using the opal reversion assay as described by Kunkel et al. (1987, Proc. Natl Acad. Sci. USA 84: 4865-4869).

The polypeptide of the invention may comprise additional functional and structural domains, for example, an affinity purification tag (such as an His purification tag), or DNA polymerase activity-enhancing domains such as the proliferating cell nuclear antigen homologue from *Archaeoglobus fulgidus*, T3 DNA polymerase thioredoxin binding domain, DNA binding protein Sso7d from *Sulfolobus solfataricus*, Sso7d-like proteins, or mutants thereof, or helix-hairpin-helix motifs derived from DNA topoisomerase V. The DNA polymerase activity-enhancing domain may also be a Cren7 enhancer domain or variant thereof, as defined and exemplified in co-pending International patent application no. PCT/GB2009/000063, which discloses that this highly conserved protein domain from Crenarchael organisms is useful to enhance the properties of a DNA polymerase, International patent application no. PCT/GB2009/000063 is incorporated herein by reference in its entirety.

In another aspect of the invention there is provided a composition comprising the polypeptide as described herein. The composition may for example include a buffer, and/or most or all ingredients for performing a reaction (such as a DNA amplification reaction for example PCR), and/or a stabiliser (such as *E. coli* GroEL protein, to enhance thermostability), and/or other compounds. The composition is in one aspect enzymatically thermostable.

The invention further provides an isolated nucleic acid encoding the polypeptide with identity to *P. helgesonii* DNA polymerase. The nucleic acid may, for example, have a sequence as shown below (5'-3'):

The non-italic underlined sequence above is outside the polymerase gene sequence and the capitalised nucleic acid "N" has been confirmed as being "A", so in a preferred embodiment the nucleic acid has the sequence shown below (5'-3'):

The nucleotide of SEQ in NO: 36 encodes the *P. helgesonii* DNA polymerase of SEQ ID NO:39 as follows:

The underlined and italicised codon "ata" coding for Isoleucine in SEQ ID NOs:2 & 36 above is a minor tRNA in *E. coli* and, therefore, this codon was changed to "att" by the inventors for expression clone work (see Henaut and Danchin (1996) in Escherichia coli and Salmonella typhimurium Cellular and Molecular Biology Vol. 2, 2047-2066, American Society for Microbiology, Washington, DC). The isolated nucleic acid having this amended nucleotide sequence is also encompassed by the invention. The altered codon does not result in any change in the expressed amino acid sequence which is also, therefore, SEQ ID NO:39.

In addition, as described in the Examples below, a "gga" motif (encoding for Glycine) was added by the inventors after the first three bases of SEQ ID NOs:2 & 36, so the first nine bases were "atgggaatt". The isolated nucleic acid variant of SEQ ID NOs:2 & 36, incorporating these changes, is encompassed by the invention, as is the isolated protein having the amino acid sequence encoded by the variants. The "gga" codon was added to introduce an *Nco*I restriction enzyme recognition sequence.

Also encompassed by the invention are further variants of the nucleic acids, as defined below.

Further provided is a vector comprising the isolated nucleic acid as described herein.

Additionally provided is a host cell transformed with the vector of the invention.

Also provided is a method for of producing a DNA polymerase of the invention comprising culturing the host cell defined herein under conditions suitable for expression of the DNA polymerase.

A recombinant polypeptide expressed from the host cell is also encompassed by the invention.

In another aspect of the invention there is provided a kit comprising the polypeptide as described herein, and/or the composition as described herein, and/or the isolated nucleic acid as described herein, and/or the vector as described herein, and/or the host cell as described herein, together with packaging materials therefor. The kit may, for example, comprise components including the polypeptide for carrying out a reaction requiring DNA polymerase activity, such as PCR.

The invention further provides a method of amplifying a sequence of a target nucleic acid using a thermocycling reaction, for example PCR, comprising the steps of:
(1) contacting the target nucleic acid with the polypeptide having thermostable DNA polymerase activity or the composition as described herein; and
(2) incubating the target nucleic acid with the polypeptide or the composition under thermocycling reaction conditions which allow amplification of the target nucleic acid.

The present invention also encompasses variants of the polypeptide as defined herein. As used herein, a "variant" means a polypeptide in which the amino acid sequence differs from the base sequence from which it is derived in that one or more amino acids within the sequence are substituted for other amino acids. Amino acid substitutions may be regarded as "conservative" where an amino acid is replaced with a different amino acid with broadly similar properties. Non-conservative substitutions are where amino acids are replaced with amino acids of a different type.

By "conservative substitution" is meant the substitution of an amino acid by another amino acid of the same class, in which the classes are defined as follows:

| Class | Amino acid examples |
|---|---|
| Nonpolar: | A, V, L, I, P, M, F, W |
| Uncharged polar: | G, S, T, C, Y, N, Q |
| Acidic: | D, E |
| Basic: | K, R, H. |

As is well known to those skilled in the art, altering the primary structure of a peptide by a conservative substitution may not significantly alter the activity of that peptide because the side-chain of the amino acid which is inserted into the sequence may be able to form similar bonds and contacts as the side chain of the amino acid which has been substituted out. This is so even when the substitution is in a region which is critical in determining the peptide's conformation.

Non-conservative substitutions are possible provided that these do not interrupt with the function of the DNA binding domain polypeptides.

Broadly speaking, fewer non-conservative substitutions will be possible without altering the biological activity of the polypeptides.

Determination of the effect of any substitution (and, indeed, of any amino acid deletion or insertion) is wholly within the routine capabilities of the skilled person, who can readily determine whether a variant polypeptide retains the thermostable DNA polymerase activity according to the invention. For example, when determining whether a variant of the polypeptide falls within the scope of the invention, the skilled person will determine whether the variant retains enzyme activity (i.e., polymerase activity) at least 60%, preferably at least 70%, more preferably at least 80%, yet more preferably 90%, 95%, 96%, 97%, 98%, 99% or 100% of the non-variant polypeptide. Activity may be measured by, for example, any standard measure such as the number of bases of a template sequence which can be replicated in a given time period.

Variants of the polypeptide may comprise or consist essentially of an amino acid sequence with at least 78% identity, for example at least 79%, 81%, 82%, 83%, 84%, 86%, 87%, 88%, 89% 91%, 92%, 93%, 94%, 96%, 97%, 98% or 99% identity to SEQ ID NO:1.

Using the standard genetic code, further nucleic acids encoding the polypeptides may readily be conceived and manufactured by the skilled person. The nucleic acid may be DNA or RNA and, where it is a DNA molecule, it may for example comprise a cDNA or genomic DNA.

The invention encompasses variant nucleic acids encoding the polypeptide of the invention. The term "variant" in relation to a nucleic acid sequences means any substitution of, variation of, modification of, replacement of deletion of, or addition of one or more nucleic acid(s) from or to a polynucleotide sequence providing the resultant polypeptide sequence encoded by the polynucleotide exhibits at least the same properties as the polypeptide encoded by the basic sequence. The term therefore includes allelic variants and also includes a polynucleotide which substantially hybridises to the polynucleotide sequence of the present invention. Such hybridisation may occur at or between low and high stringency conditions. In general terms, low stringency conditions can be defined a hybridisation in which the washing step takes place in a 0.330-0.825 M NaCl buffer solution at a temperature of about 40-48°C below the calculated or actual melting temperature (Tₘ) of the probe sequence (for example, about ambient laboratory temperature to about 55°C), while high stringency conditions involve a wash in a 0.0165-0.0330 M NaCl buffer solution at a temperature of about 5-10°C below the calculated or actual Tₘ of the probe(for example, about 65°C). The buffer solution may, for example, be SSC buffer (0.15M NaCl and 0.015M tri-sodium citrate), with the low stringency wash taking place in 3 x SSC buffer and the high stringency wash taking place in 0.1 x SSC buffer. Steps involved in hybridisation of nucleic acid sequences have been described for example in Sambrook et al. (1989; Molecular Cloning, Cold Spring Harbor Laboratory Press, Cold Spring Harbor).

Typically, variants have 77% or more of the nucleotides in common with the nucleic acid sequence of the present invention, for example 78%, 79%, 80%, 81%, 82%, 83%, 84%, 85%, 86%, 87%, 88%, 89%, 90%, 91%, 92%, 93%, 94%, 95%, 96%, 97%, 98% or 99% or greater sequence identity.

Variant nucleic acids of the invention may be codon-optimised for expression in a particular host cell.

DNA polymerases and nucleic acids of the invention may be prepared synthetically using conventional synthesisers. Alternatively, they may be produced using recombinant DNA technology or isolated from natural sources followed by any chemical modification, if required. In these cases, a nucleic acid encoding the chimeric protein is incorporated into suitable expression vector, which is then used to transform a suitable host cell, such as a prokaryotic cell such as *E. coli.* The transformed host cells are cultured and the protein isolated therefrom. Vectors, cells and methods of this type form further aspects of the present invention.

Sequence identity between nucleotide and amino acid sequences can be determined by comparing an alignment of the sequences. When an equivalent position in the compared sequences is occupied by the same amino acid or base, then the molecules are identical at that position. Scoring an alignment as a percentage of identity is a function of the number of identical amino acids or bases at positions shared by the compared sequences. When comparing sequences, optimal alignments may require gaps to be introduced into one or more of the sequences to take into consideration possible insertions and deletions in the sequences. Sequence comparison methods may employ gap penalties so that, for the same number of identical molecules in sequences being compared, a sequence alignment with as few gaps as possible, reflecting higher relatedness between the two compared sequences, will achieve a higher score than one with many gaps. Calculation of maximum percent identity involves the production of an optimal alignment, taking into consideration gap penalties.

In addition to the BLASTP computer program mentioned above, further suitable computer programs for carrying out sequence comparisons are widely available in the commercial and public sector. Examples include the MatGat program (Campanella et al., 2003, BMC Bioinformatics 4: 29), the Gap program (Needleman & Wunsch, 1970, J. Mol. Biol. 48: 443-453) and the FASTA program (Altschul et al., 1990, J. Mol. Biol. 215: 403-410). MatGAT v2.03 is freely available from the website "http://bitincka.com/ledion/matgat/" (accessed 12 March 2009) and has also been submitted for public distribution to the Indiana University Biology Archive (IUBIO Archive). Gap and FASTA are available as part of the Accelrys GCG Package Version 11.1 (Accelrys, Cambridge, UK), formerly known as the GCG Wisconsin Package. The FASTA program can alternatively be accessed publicly from the European Bioinformatics Institute (http://www.ebi.ac.uk/fasta, accessed 12 March 2009) and the University of Virginia (http://fasta.biotech.virginia.edu/fasta www/cgi or http://fasta.bioch.virginia.edu/fasta_www2/fasta_list2.shtml, accessed 12 March 2009). FASTA may be used to search a sequence database with a given sequence or to compare two given sequences (see http://fasta.bioch.virginia.edu/fasta_www/cgi/ search_frm2.cgi, accessed 12 March 2009). Typically, default parameters set by the computer programs should be used when comparing sequences. The default parameters may change depending on the type and length of sequences being compared. A sequence comparison using the MatGAT program may use default parameters of Scoring Matrix = Blosum50, First Gap = 16, Extending Gap = 4 for DNA, and Scoring Matrix = Blosum50, First Gap = 12, Extending Gap = 2 for protein. A comparison using the FASTA program may use default parameters of Ktup = 2, Scoring matrix = Blosum50, gap = -10 and ext = -2.

In one aspect of the invention, sequence identity is determined using the MatGAT program v2.03 using default parameters as noted above.

As used herein, a "DNA polymerase" refers to any enzyme that catalyzes polynucleotide synthesis by addition of nucleotide units to a nucleotide chain using a nucleic acid such as DNA as a template. The term includes any variants and recombinant functional derivatives of naturally occurring nucleic acid polymerases, whether derived by genetic modification or chemical modification or other methods known in the art.

As used herein, "thermostable" DNA polymerase activity means DNA polymerase activity which is relatively stable to heat and functions at high temperatures, for example 45-100°C, preferably 55-100°C, 65-100°C, 75-100°C, 85-100°C or 95-100°C, as compared, for example, to a non-thermostable form of DNA polymerase.

### Brief description of Figures

Particular non-limiting embodiments of the present invention will now be described with reference to the following Figures, in which:

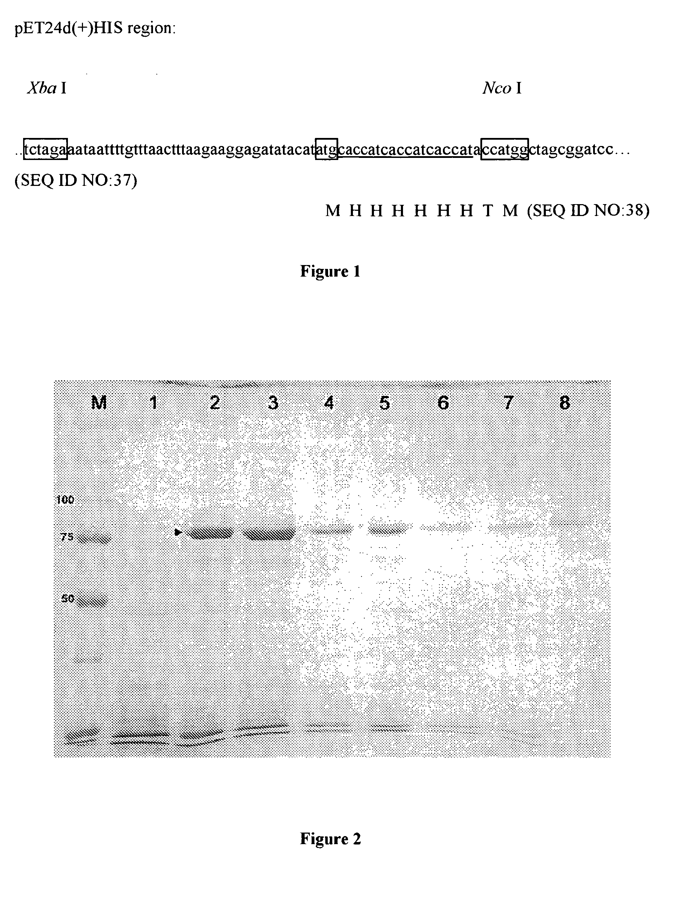

Figure 1 is a diagram showing the structure of the pET24d(+)HIS region used in cloning of a *Palaeococcus helgesonii* DNA polymerase according to a first embodiment of the invention;
Figure 2 is an SDS PAGE gel of fractionated expressed *Palaeococcus helgesonii* DNA polymerase according to the first embodiment of the invention referred to in Figure 1. Lane M is a Bio-Rad Precision Plus Protein Standard; lane 1 is induced negative control (equivalent of 100µl *E. coli*); lane 2 is induced *P. helgesonii* DNA polymerase-containing clone (equivalent of 50µl *E. coli*); lane 3 is induced HIS-tagged *P. helgesonii* DNA polymerase-containing clone (equivalent of 50µl *E. coli*); lane 4 is induced *P. helgesonii* DNA polymerase-containing clone (equivalent of 12.5µl *E. coli*); lane 5 is induced HIS-tagged *P. helgesonii* DNA polymerase-containing clone (equivalent of 12.5µl *E. coli*); lane 6 is induced *P. helgesonii* DNA polymerase-containing clone (equivalent of 5µl *E. coli*); lane 7 is induced HIS-tagged *P. helgesonii* DNA polymerase-containing clone (equivalent of 5µl *E. coli*); and lane 8 is 25u Pfu polymerase; and

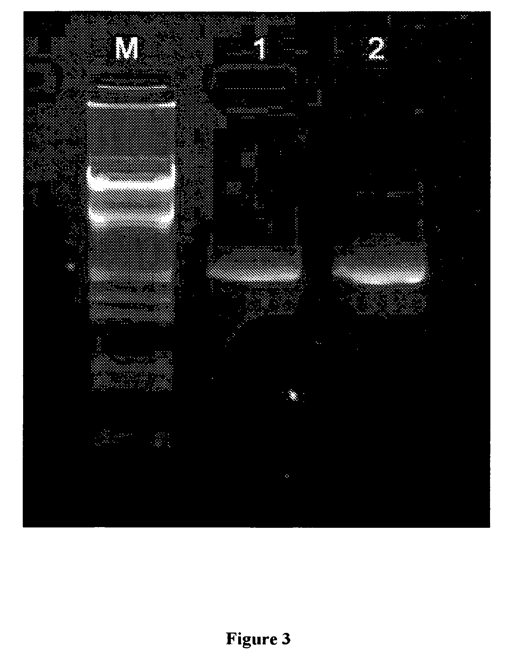

Figure 3 is an agarose gel of fractionated PCR reaction samples following amplification of lambda (λ) DNA using the *Palaeococcus helgesonii* DNA polymerase according to the first embodiment of the invention referred to in Figures 1 and 2. Lane M is an *Eco*R I/*Hin*d III Lambda DNA marker (band sizes (in bp):564, 831, 947, 1375, 1584, 1904, 2027, 3530, 4268, 4973, 5148, 21226); lane 1 is a PCR sample amplified using 1.25u Pfu polymerase (positive control); and lane 2 is a PCR sample amplified using 2.5µl of an *E. coli* extract of a *P. helgesonii* DNA polymerase-containing clone (non-HIS tagged).

### Examples

Lyophilized cultures of *Palaeococcus helgesonii* were obtained from the Deutsche Sammlung von Mikroorganismen und Zellkulturen GmbH (German Collection of Microorganisms and Cell Cultures; Accession No. DSM 15127). As described below, following extraction and amplification of gDNA from the cultures, a gene walking method was used, as outlined below, to reach the predicted 5' start and the 3' stop of a putative DNA polymerase B gene ("DNA polB") encoding a putative DNA polymerase II.

### Example 1 Genomic DNA extraction

The method for genomic DNA extraction from *P. helgesonii* cultures was derived from Gotz et al. (2002; Int. J. Syst. Evol. Microbiol. 52: 1349-1359) which is a modification of a method described in Ausubel et al. (1994; Current Protocols in Molecular Biology, Wiley, New York).

Cell pellets were resuspended in 567µl 1x TE buffer (10mM Tris/HCl, pH8.0; 1mM EDTA), 7.5% Chelex 100 (Sigma), 50mM EDTA (pH7.0), 1% (w/v) SDS and 200µg Proteinase K and incubated with slow rotation for 1h at 50°C. Chelex was removed by centrifugation. Then 100µl 5M NaCl and 80µl 10% (w/v) cetyltrimethylammonium bromide in 0.7M NaCl were added to the cell lysate and the sample incubated for 30 mins at 65°C. The DNA was extracted with phenol/chloroform, isopropanol precipitated and the DNA resuspended in water. DNA concentration was estimated on a 1% agarose gel.

### Example 2 Initial screening for putative DNA polB gene

The screening method was derived from Shandilya et al. (2004, Extremophiles 8: 243-251) and Griffiths et al. (2007, Protein Expression & Purification 52:19-30).

Using degenerate Pol primers ARCHPOLR1 and ARCHPOLF1 (see below), a -730bp fragment was amplified from 10ng *P. helgesonii* gDNA.

The ARCHPOLR1 primer has the sequence:
5'-CGC GGG AGA ACC TGG TTN TCD ATR TAR TA-3' (SEQ ID NO:3) (corresponding to the amino acid sequence YYIENQVLP, SEQ ID NO:4); and
the ARCHPOLF1 primer has the sequence:
5'-TAC TAC GGA TAG GCC AAR GCN AGR TGG TA-3' (SEQ ID NO:5) (corresponding to the amino acid sequence YYGXAKARW, SEQ ID NO:6).

"X" in SEQ ID NO:6 represents a "STOP" codon, as derived from the primer sequence which is as used by Griffiths *et al.* The primer is still effective in this gene walking method as demonstrated in the present application and also by the work of Griffiths *et al.*

The PCR reaction mix was as follows:

| | |
|---|---|
| 10x PCR Buffer (750mM Tris-HCl, pH8.8, 200mM (NH₄)₂SO₄, 0.1% (v/v) Tween-20) | 10µl |
| 5mM dNTP's | 2µl |
| 5' primer (10pM/µl) | 2.5µl |
| 3' primer (10pM/µl) | 2.5µl |
| gDNA | 10ng |
| *Taq* DNA Polymerase (5u/µl) | 0.25µl |
| Water | To 50µl. |

PCR cycling conditions were 4 minute initial denaturation at 94°C followed by 15 cycles of: 10 seconds denaturation at 94°C, 30 seconds annealing at 60°C (reducing by 1°C per cycle), 1 minute extension at 72°C. This was followed by a further step of 35 cycles of: 10 seconds denaturation at 94°C, 10 seconds annealing at 55°C, 1 minute extension at 72°C. Final extension at 72°C for 7 mins. 4°C hold.

A ~730bp amplified product was TA cloned (Invitrogen pCR2.1 kit. Cat#K2000-01) and sequenced using M13 Forward (5'-TGT AAA ACG ACG GCC AGT-3', SEQ NO:7) and Reverse (5'-AGCGGATAACAATTTCACACAGGA-3', SEQ ID NO:8) primers on an ABI-3100 DNA sequencer. Sequencing data confirmed the fragment was of a putative PolB gene.

Sequence data were aligned with that of previously published DNA polymerase DNA sequence data (*P. wo*, *P.fu*, *P.gl*, *P.spGE23*, *P.ab*, *P.spST700*, *T.on*, *T.spGE8*, *T.zi*, *T.spGT*, *T.hy*, *T.th*, *T.spTY*, *T.li*, *T.sp9N7*, *T.fu*) and a new primer (15127_1) was designed.

The 15127_1 primer has the sequence:
5' - CAT CCA CAG GAC GTC CC - 3' (SEQ ID NO:9)
(corresponding to the amino acid sequence HPQDVP, SEQ ID NO:10).

A specific lower primer 15127_L1 (5'-TAAACCCGAATCCCTCTGCC-3', SEQ ID NO:11) was designed and used in PCR with 15127_1 to amplify a ~1340bp fragment.

The PCR reaction mix was as follows:

| | |
|---|---|
| 10x PCR Buffer (750mM Tris-HCl, pH8.8, 200mM (NH₄)₂SO₄, 0.1% (v/v) Tween-20) | 5µl |
| 5mM dNTP's | 2µl |
| 5' primer (10pM/µl) | 2.5µl |
| 3' primer (10pM/µl) | 2.5µl |
| gDNA | 10ng |
| *Taq* DNA Polymerase (5u/µl) | 0.25µl |
| Water | To 50µl. |

PCR cycling conditions were 4 minute initial denaturation at 94°C followed by 15 cycles of: 10 seconds denaturation at 94°C, 10 seconds annealing at 60°C (reducing by 1°C per cycle), 2 minute extension at 72°C. This was followed by a further step of 35 cycles of: 10 seconds denaturation at 94°C, 10 seconds annealing at 55°C, 2 minute extension at 72°C. Final extension was at 72°C for 7mins. 4°C hold.

A ~1340bp amplified product was ExoSAP treated and sequenced using primer 15127_L1, and later 15127_L2 (5'-TTGTGTGCCTCACGACGGGA-3', SEQ ID NO:12).

### Example 3 Gene walking

From the amplification product obtained in Example 2, primers were designed to walk along' *P. helgesonii* gDNA to reach the 5' start (N-terminus of gene product) and 3' stop (C-terminus of gene product) of the putative DNA polB gene.

10ng gDNA was digested individually with 5u of various 6 base pair-cutter restriction endonucleases in 10µl reaction volume and incubated for 3 h at 37°C. 12 individual digest reactions were run, using a unique 6-cutter restriction enzyme (RE) for each. 5µl digested template was then self-ligated using 12.5u T4 DNA Ligase, 1µl 10x ligase buffer in 50µl reaction volume, with an overnight incubation at 16°C.

Self-ligated DNA was then used as template in two rounds of PCR, the second of which used nested primers to give specificity to amplification.

### C-terminus end:

Primers were designed from the ~730bp sequenced fragment to 'walk' to the end of the DNA polymerase gene.

The primers were:
15127_C-ter_Upper (5'-GCAAGGGGCATCAAGGTGAAGC-3') SEQ ID NO:13
15127_C-ter_Upper_Nested (5'-TGTTTTGAAGGGGAGCAAGAAG-3') SEQ ID NO:14
15127_C-ter_Lower (5'-GCTTTTCTACGGGAACGCGGTA-3') SEQ ID NO:15
15127_C-ter_Lower_Nested (5'-GTGACGCTCTCGGCACACTC-3') SEQ ID NO:16.

### First round PCR:

The PCR reaction mix was as follows:

| | |
|---|---|
| Self-ligation reaction (~100pg/µl DNA) | 2µl |
| 10x PCR Buffer (200mM Tris-HCl, pH8.8, 100mM KCl, 100mM (NH₄)₂SO₄, 1% (v/v) Triton X-100, 20mM MgSO₄) | 5 µl |
| 5mM dNTP's | 2µl |
| 15127_C-ter_Upper | 25pM |
| 15127_C-ter_Lower | 25pM |
| Taq/Pfu (20:1) (5u/µl) | 1.25u |
| Water | To 50µl. |

Cycling conditions were 4 minute initial denaturation at 94°C followed by 35 cycles of: 10 seconds denaturation at 94°C, 10 seconds annealing at 55°C, 5 minute extension at 72°C. Final extension was at 72°C for 7mins. 4°C hold.

### Second round (nested) PCR:

The PCR reaction mix was as follows:

| | |
|---|---|
| 1^{st} round PCR reaction | 1µl |
| 10x PCR Buffer (200mM Tris-HCl, pH8.8, 100mM KCl, 100mM (NH₄)₂SO₄, 1% (v/v) Triton X-100, 20mM MgSO₄) | 5µl |
| 5mM dNTP's | 2µl |
| 15127_C-ter_Upper_Nested | 25pM |
| 15127_C-ter_Lower_Nested | 25pM |
| Taq/Pfu (20:1) (5u/µl) | 1.25u |
| Water | To 50µl. |

Cycling conditions were 4 minute initial denaturation at 94°C followed by 25 cycles of: 10 seconds denaturation at 94°C, 10 seconds annealing at 55°C, 5 minute extension at 72°C. Final extension was at 72°C for 7mins. 4°C hold.

PCR fragments between ∼0.5kb and ∼2.5kb were obtained from *Nco* I, *Hind* III, *Nhe* I, *Fsp* I digested/self-ligated reaction templates.

These fragments were sequenced using the nested primers. Sequencing of fragments indicated that the C-terminal STOP codon of the DNA polymerase gene had been reached.

### N-terminus end:

Primers were designed from the ~1340bp sequenced fragment to 'walk' to the start of the DNA polymerase gene.

These primers were:
15127_N-ter_Lower_Nested (5'-CCACAACGGCAGGATGGTTC-3') SEQ ID NO:17
15127_N-ter_Lower (5'-TAGATGTCCACAACGGCAGG-3') SEQ ID NO:18
15127_N-ter_Upper (5'-CAGAGGGCTTGTTCCGATGG-3') SEQ ID NO:19.

### First round PCR:

The PCR reaction mix was as follows:

| | |
|---|---|
| Self-ligation reaction (∼100pg/µl DNA) | 2µl |
| 10x PCR Buffer (200mM Tris-HCl, pH8.8, 100mM KCl, 100mM (NH₄)₂SO₄, 1% (v/v) Triton X-100, 20mM MgSO₄) | 5µl |
| 5mM dNTP's | 2µl |
| 15127_N-ter_Upper | 25pM |
| 15127_N-ter_Lower | 25pM |
| Taq/Pfu (20:1) (5u/µl) | 1.25u |
| Water | To 50µl. |

Cycling conditions were 4 minute initial denaturation at 94°C followed by 35 cycles of: 10 seconds denaturation at 94°C, 10 seconds annealing at 55°C, 5 minute extension at 72°C. Final extension was at 72°C for 7mins. 4°C hold.

### Second round (nested) PCR:

The PCR reaction mix was as follows:

| | |
|---|---|
| 1^{st} round PCR reaction | 1µl |
| 10x PCR Buffer (200mM Tris-HCl, pH8.8, 100mM KCI, 100mM (NH₄)₂SO₄, 1% (v/v) Triton X-100, 20mM MgSO₄) | 5µl |
| 5mM dNTP's | 2µl |
| 15127_N-ter_Upper | 25pM |
| 15127_N-ter_Lower_Nested | 25pM |
| Taq/Pfu (20:1) (5u/µl) | 1.25u |
| Water | To 50µl. |

Cycling conditions were 4 minute initial denaturation at 94°C followed by 25 cycles of: 10 seconds denaturation at 94°C, 10 seconds annealing at 55°C, 5 minute extension at 72°C. Final extension was at 72°C for 7mins. 4°C hold.

PCR fragments between ∼0.5kb and ∼3.5kb were obtained from *Nco I*, *Nde I*, *Nsi I*, *Xho I* digested/self-ligated reaction templates.

These fragments were sequenced using the nested round primers. Sequencing of the fragments showed that the N-terminal ATG start codon had been reached.

### Example 4 Amplification of DNA polymerase gene

The gene walking protocols described in Example 3 reached the predicted start and stop of the DNA polymerase (polB) gene. Specific primers were designed to amplify the ∼2.3kb full length gene (as determined by alignments with previously reported DNA polymerases such as *Pfu*).

Restriction sites (underlined) were built into primers to allow easy cloning into vectors.

The primers were:
15127_FL_Start_(*Nco*I)
5'-AAGCTTCCATGGGTATTCTTGATACAGATTATATAACGGA-3' (SEQ ID NO:20)
15127_STOP_(*Sal*I)
5'-GGATCCGTCGACTTACCCCTTCCCCGTTCCAAGCCACGC-3' (SEQ ID NO:21);

Gene products were amplified using a high fidelity Phusion DNA polymerase (New England Biolabs).

The PCR solution consisted of:

| | |
|---|---|
| 5x HF Phusion reaction Buffer | 20µl |
| 5mM dNTP's | 4µl |
| 5' primer [15127_FL_Start_(*Nco*I)] | 25pM |
| 3' primer [15127_STOP_(*Sal*I)] | 25pM |
| gDNA | 10ng |
| Phusion DNA Polymerase (2u/µl) | 0.5µl |
| Water | To 100µl. |

Cycling conditions were: 30 seconds initial denaturation at 98°C followed by 25 cycles of: 3 seconds denaturation at 98°C, 10 seconds annealing at 55°C, 2.5 minute extension at 72°C. Final extension was at 72°C for 7mins. 4°C hold.

### Example 5 pET24d(+)HIS vector construction

The pET24d(+) vector (Novagen) was modified to add a 6x HIS tag upstream of *Nco*I site (see Figure 1). The HIS tag was inserted between *Xba*I and *BamH*I sites as follows.

An overlapping primer pair, of which an upper primer (*Xba*I) has the sequence: a lower primer (*BamH*I) has the sequence: were amplified by PCR, RE digested and ligated into pET24d(+). The ligation reaction was transformed into *E. coli* TOP10F' (Invitrogen) and plated on Luria Broth plates plus kanamycin. Colonies were screened by PCR and verified by sequencing using T7 sequencing primers:
T7_Promoter: 5'-AAATTAATACGACTCACTATAGGG-3' (SEQ ID NO:24)
T7_Terminator: 5'-GCTAGTTATTGCTCAGCGG-3' (SEQ ID NO:25)

### Example 6 Cloning of DNA polymerase

The ∼3.9kb fragment PCR product from Example 4 was purified using Promega Wizard purification kit and then RE digested using *Nco* I/*Sal* I. DNA was phenol/chloroform extracted, ethanol-precipitated and resuspended in water. The fragment was then ligated into pET24d(+) and pET24d(+)HIS, between *Nco* I and *Sal* I, and electroporated into KRX cells (Promega). Colonies were screened by PCR using vector-specific T7 primers. The KRX (pRARE2) cell strain was produced by electroporating the pRARE2 plasmid (isolated from Rosetta2 [EMD Biosciences]) into *E.coli* KRX (Promega). The pRARE2 plasmid supplies tRNAs for seven rare codons (AUA, AGG, AGA, CUA, CCC, CGG, and GGA) on a chloramphenicol-resistant plasmid.

### Example 7 Expression of DNA polymerase

Recombinant colonies from Example 6 were grown up overnight in 5 ml Luria Broth (including Kanamycin/Chloramphenicol). 50 ml Terrific Broth baffled shake flasks were inoculated by 1/100 dilution of overnight culture. Cultures were grown at 37°C, 275 rpm to OD₆₀₀∼1 then brought down to 24°C and induced with L-rhamnose to 0.1% final concentration, and IPTG to 10mM final concentration. Cultures were incubated for a further 18h at 24°C, 275rpm. 10ml of the culture was then harvested by centrifugation for 10mins at 5,000xg and cells were resuspended in 1ml Lysis buffer (50mM Tris-HCl, pH8.0, 100mM NaCl, 1mM EDTA) and sonicated for 2 bursts of 30s (40v) on ice. Samples were centrifuged at 5,000xg for 5min and heat lysed at 70°C for 20min to denature background *E.coli* proteins. Samples were centrifuged and aliquots of supernatant were size fractionated on 8% SDS-PAGE.

Expressed protein bands were visible at the expected molecular weight of ∼90kDa, as shown in Figure 2.

### Example 8 PCR activity assay

PCR activity of the samples obtained in Example 7 was tested in a 2kb λDNA PCR assay. *Pfu* DNA polymerase (1.25 u) was used as positive control.

The PCR solution contained:

| | |
|---|---|
| 10x PCR Buffer (750mM Tris-HCl, pH8.8, 200mM (NH₄)₂SO₄, 0.1% (v/v) Tween-20) | 5µl |
| 5mM dNTP mix | 2µl |
| Enzyme test sample | 1µl |
| Upper λ primer | 25pM |
| Lower λ primer | 25pM |
| λDNA | 1ng |
| Water | To 50µl. |

The Upper λ primer has the sequence:
5' - CCTGCTCTGCCGCTTCACGC - 3' (SEQ ID NO:26),
while the Lower primer has the sequence:
5' - CCATGATTCAGTGTGCCCGTCTGG - 3' (SEQ ID NO:27).

PCR proceeded with 35 cycles of: 3 seconds denaturation at 94°C, 10 seconds annealing at 55°C, 2 minutes extension at 72°C. Final extension at 72°C for 7 mins. 4°C hold.

Aliquots of the reaction products were run out on a 1% agarose gel, and the P. *helgesonii* DNA polymerase was found to amplify the expected 2 kb λ DNA fragment as shown in Figure 3.

## Claims

1. A polypeptide having thermostable DNA polymerase activity and comprising or consisting of an amino acid sequence with at least 79% identity to *Palaeococcus helgesonii* DNA polymerase shown in SEQ ID NO:1.

2. The polypeptide according to claim 1 comprising or consisting of an amino acid sequence with at least 78% identity to *Palaeococcus helgesonii* DNA polymerase shown in SEQ ID NO:39.

3. The polypeptide according to claim 1 or 2, which is suitable for carrying out a thermocycling amplification reaction, such as a polymerase chain reaction (PCR).

4. The polypeptide according to any of claims 1-3, in which the polypeptide has 3'-5' exonuclease proofreading activity.

5. The polypeptide according to any preceding claim, in which the polypeptide lacks 5'-3'exonuclease activity.

6. The polypeptide according to any preceding claim, which is an isolated thermostable DNA polymerase obtainable from *Palaeococcus helgesonii* and having a molecular weight of about 90,000 Daltons, or a fragment thereof having thermostable DNA polymerase activity.

7. A polypeptide according to claim 1 having thermostable DNA polymerase activity and comprising the amino acid sequence SEQ ID NO:39.

8. A polypeptide according to any of claims 1-7, further comprising a Cren7 enhancer domain.

9. A composition comprising the polypeptide of any of claims 1-8.

10. The composition according to claim 9, which is enzymatically thermostable.

11. A nucleic acid encoding the polypeptide of any of claims 1-7, for example having a nucleotide sequence shown in SEQ ID NO: 36, or encoding the polypeptide of claim 8.

12. A vector comprising the nucleic acid of claim 11.

13. A host cell transformed with the vector of claim 12.

14. A kit comprising the polypeptide of any of claims 1-8, and/or the composition of claim 9 or claim 10, and/or the nucleic acid of claim 11, and/or the vector of claim 12, and/or the host cell of claim 13, together with packaging materials therefor.

15. A method of amplifying a sequence of a target nucleic acid using a thermocycling reaction, comprising the steps of:
(1) contacting the target nucleic acid with the polypeptide of any of claims 1-8, and/or the composition of either of claim 9 or claim 10; and
(2) incubating the target nucleic acid with the polypeptide and/or composition under thermocycling reaction conditions which allow amplification of the target nucleic acid.

## Patentansprüche

1. Polypeptid, das eine thermostabile DNA-Polymerase-Aktivität besitzt und das eine Aminosäuresequenz mit mindestens 79 % Identität mit *Palaeococccrs helgesonii*-DNA-Polymerase wie in SEQ ID NO:1 dargestellt umfasst oder aus einer solchen besteht.

2. Polypeptid gemäß Anspruch 1, das eine Aminosäuresequenz mit mindestens 78 % Identität mit *Palaeococcus helgesonii*-DNA-Polymerase wie in SEQ ID NO:39 dargestellt umfasst oder aus einer solchen besteht.

3. Polypeptid gemäß Anspruch 1 oder 2, das zur Durchführung einer thermozyklischen Amplifizierungsreaktion geeignet ist, wie einer Polymerase-Kettenreaktion (PCR).

4. Polypeptid gemäß einem der Ansprüche 1 bis 3, in dem das Polypeptid eine 3'-5'-Exo-nuklease-Proofreading-Aktivität aufweist.

5. Polypeptid gemäß einem der vorangehenden Ansprüche, in dem dem Polypeptid eine 5'-3'-Exonuklease-Aktivität fehlt.

6. Polypeptid gemäß einem der vorangehenden Ansprüche, das eine isolierte thermostabile, von *Palaeococcus helgesonii* erhältliche DNA-Polymerase mit einem Molekulargewicht von etwa 90.000 Dalton oder ein Fragment davon mit thermostabiler DNA-Polymerase-Aktivität ist.

7. Polypeptid gemäß Anspruch 1, das eine thermostabile DNA-Polymerase-Aktivität aufweist und die Aminosäuresequenz SEQ ID NO:39 umfasst.

8. Polypeptid gemäß einem der Ansprüche 1 bis 7, das ferner eine Cren7-Enhancer-Domain umfasst.

9. Zusammensetzung, die das Polypeptid eines der Ansprüche 1 bis 8 umfasst.

10. Zusammensetzung gemäß Anspruch 9, die enzymatisch thermostabil ist.

11. Nukleinsäure, die das Polypeptid eines der Ansprüche 1 bis 7 codiert, beispielsweise mit einer in SEQ ID NO:36 dargestellten Nukleotidsequenz, oder die das Polypeptid nach Anspruch 8 codiert.

12. Vektor, der die Nukleinsäure nach Anspruch 11 umfasst.

13. Wirtszelle, die mit dem Vektor nach Anspruch 12 transformiert ist.

14. Kit, das das Polypeptid nach einem der Ansprüche 1 bis 8 und/oder die Zusammensetzung nach Anspruch 9 oder Anspruch 10 und/oder die Nukleinsäure nach Anspruch 11 und/oder den Vektor nach Anspruch 12 und/oder die Wirtszelle nach Anspruch 13 zusammen mit Verpackungsmaterialien hierfür umfasst.

15. Verfahren zur Amplifizierung einer Sequenz einer Target-Nukleinsäure unter Anwendung einer thermozyklischen Reaktion, folgende Schritte umfassend:
(1) Kontaktierten der Target-Nukleinsäure mit dem Polypeptid eines der Ansprüche 1 bis 8 und/oder der Zusammensetzung nach Anspruch 9 oder Anspruch 10; und
(2) Inkubieren der Target-Nukleinsäure mit dem Polypeptid und/oder der Zusammensetzung unter thermozyklischen Reaktionsbedingungen, die die Amplifizierung der Target-Nukleinsäure erlauben.

## Revendications

1. Polypeptide ayant une activité ADN polymérase thermostable et comprenant ou consistant en une séquence d'acides aminés présentant au moins 79 % d'identité avec l'ADN polymérase de *Palaeococcus helgesonii* représentée par SEQ ID NO : 1.

2. Polypeptide selon la revendication 1 comprenant ou consistant en une séquence d'acides aminés présentant au moins 78 % d'identité avec l'ADN polymérase de *Palaeococcus helgesonii* représentée par SEQ ID NO : 39.

3. Polypeptide selon la revendication 1 ou 2, qui est approprié pour la réalisation d'une réaction d'amplification avec thermocyclage, telle qu'une réaction en chaîne de la polymérase (PCR).

4. Polypeptide selon l'une quelconque des revendications 1 à 3, dans lequel le polypeptide a une activité de relecture d'exonucléase 3'-5'.

5. Polypeptide selon l'une quelconque des revendications précédentes, dans lequel le polypeptide manque d'activité exonucléase 5'-3'.

6. Polypeptide selon l'une quelconque des revendications précédentes, qui est une ADN polymérase thermostable isolée pouvant être obtenue à partir de *Palaeococcus helgesonii* et ayant une masse moléculaire d'environ 90 000 Daltons, ou un fragment de celle-ci ayant une activité ADN polymérase thermostable.

7. Polypeptide selon la revendication 1 ayant une activité ADN polymérase thermostable et comprenant la séquence d'acides aminés SEQ ID NO : 39.

8. Polypeptide selon l'une quelconque des revendications 1 à 7, comprenant en outre un domaine amplificateur de Cren7.

9. Composition comprenant le polypeptide selon l'une quelconque des revendications 1 à 8.

10. Composition selon la revendication 9, qui est thermostable d'un point de vue enzymatique.

11. Acide nucléique codant pour le polypeptide selon l'une quelconque des revendications 1 à 7, par exemple ayant une séquence nucléotidique représentée par SEQ ID NO : 36, ou codant pour le polypeptide selon la revendication 8.

12. Vecteur comprenant l'acide nucléique selon la revendication 11.

13. Cellule hôte transformée avec le vecteur selon la revendication 12.

14. Kit comprenant le polypeptide selon l'une quelconque des revendications 1 à 8, et / ou la composition selon la revendication 9 ou la revendication 10, et/ou l'acide nucléique selon la revendication 11, et/ou le vecteur selon la revendication 12, et/ou la cellule hôte selon la revendication 13, conjointement avec des matériaux de conditionnement de ceux-ci.

15. Procédé d'amplification d'une séquence d'un acide nucléique cible en utilisant une réaction de thermocyclage, comprenant les étapes suivantes :
(1) la mise en contact de l'acide nucléique cible avec le polypeptide selon l'une quelconque des revendications 1 à 8, et/ou la composition selon l'une ou l'autre des revendications 9 et 10 ; et
(2) l'incubation de l'acide nucléique cible avec le polypeptide et/ou la composition dans des conditions de réaction de thermocyclage qui permettent l'amplification de l'acide nucléique cible.


### SEQUENCE LISTING

<110> Genesys Ltd
   Clark, Duncan R
   Nicholas, Morant
<120> Enzyme
<130> P1444EP00
<140> 09718831.2
   <141> 2009-03-13
<150> GB0804721.9 <151> 2008-03-14
<150> US 61/069429 <151> 2008-03-14
<160> 39
<170> PatentIn version 3.5
<210> 1
   <211> 773
   <212> PRT
   <213> Palaeococcus helgesonii
<220>
   <221> MISC_FEATURE
   <222> (622)..(622)
   <223> Xaa is Leu, Pro, Gln or Arg
<400> 1
   Met Ile Leu Asp Thr Asp Tyr Ile Thr Glu Asn Gly Lys Pro Val Ile
   1               5                   10                  15
   Arg Ile Phe Lys Lys Glu Asn Gly Glu Phe Lys Ile Glu Tyr Asp Arg
               20                  25                  30
   Asn Phe Glu Pro Tyr Ile Tyr Ala Leu Leu Glu Asn Glu Glu Glu Ile
           35                  40                  45
   Glu Asp Ile Lys Arg Ile Thr Ala Glu Arg His Gly Lys Lys Val Arg
       50                  55                  60
   Ile Val Arg Ala Glu Lys Val Lys Lys Lys Phe Leu Gly Glu Pro Ile
   65                  70                  75                  80
   Glu Val Trp Lys Leu Val Phe Glu His Pro Gln Asp Val Pro Asp Ile
                   85                  90                  95
   Ile Arg Lys His Pro Ala Val Val Asp Ile Tyr Glu Tyr Asp Ile Pro
               100                 105                 110
   Phe Ala Lys Arg Tyr Leu Ile Asp Arg Gly Leu Val Pro Met Glu Gly
           115                 120                 125
   Asp Glu Glu Leu Lys Met Leu Ala Phe Asp Ile Glu Thr Phe Tyr His
   130 135 140
   Glu Gly Asp Glu Phe Gly Glu Gly Glu Ile Leu Met Ile Ser Tyr Ala
   145 150 155 160
   Asp Glu Gly Gly Ala Arg Val Ile Thr Trp Lys Arg Ile Asp Leu Pro
   165 170 175
   Tyr Val Glu Thr Val Ser Thr Glu Arg Glu Ala Ile Lys Arg Phe Leu
   180 185 190
   His Val Leu Lys Glu Lys Asp Pro Asp Val Leu Ile Thr Tyr Asn Gly
   195 200 205
   Asp Asn Phe Asp Phe Ala Tyr Ile Lys Lys Arg Cys Glu Lys Leu Gly
   210 215 220
   Leu Lys Phe Thr Ile Gly Arg Asp Gly Ser Glu Pro Lys Ile Gln Arg
   225 230 235 240
   Met Gly Asp Arg Phe Ala Val Glu Val Lys Gly Ile Lys Gly Arg Ile
   245 250 255
   His Leu Asp Leu Tyr Pro Val Val Arg His Thr Ile Arg Leu Pro Thr
   260 265 270
   Tyr Thr Leu Glu Ala Val Tyr Glu Ala Val Phe Gly Lys Arg Lys Glu
   275 280 285
   Lys Val Tyr Ala Glu Glu Ile Ala Thr Ala Trp Lys Ser Glu Glu Gly
   290 295 300
   Leu Lys Arg Val Ala Gln Tyr Ser Met Glu Asp Ala Lys Ala Thr Tyr
   305 310 315 320
   Glu Leu Gly Arg Glu Phe Phe Pro Met Glu Val Glu Leu Ala Lys Leu
   325 330 335
   Ile Gly Gln Ser Val Trp Asp Val Ser Arg Ser Ser Thr Gly Asn Leu
   340 345 350
   Val Glu Trp Tyr Leu Leu Arg Glu Ala Tyr Glu Arg Asn Glu Leu Ala
   355 360 365
   Pro Asn Lys Pro Gly Asp Ala Glu Tyr Arg Lys Arg Met Arg Ser Ser
   370 375 380
   Tyr Leu Gly Gly Tyr Val Lys Glu Pro Glu Lys Gly Leu Trp Glu Ser
   385 390 395 400
   Ile Ala Tyr Leu Asp Phe Arg Ser Leu Tyr Pro Ser Ile Ile Val Thr
   405 410 415
   His Asn Val Ser Pro Asp Thr Leu Glu Arg Glu Cys Lys Asn Tyr Tyr
   420 425 430
   Val Ala Pro Val Val Gly Tyr Arg Phe Cys Ser Asp Phe Lys Gly Phe
   435 440 445
   Ile Pro Ser Ile Leu Glu Glu Leu Ile Glu Thr Arg Gln Lys Val Lys
   450 455 460
   Arg Lys Met Lys Ala Thr Ile Asp Pro Val Glu Arg Lys Met Leu Asp
   465 470 475 480
   Tyr Arg Gln Arg Ala Leu Lys Ile Leu Ala Asn Ser Tyr Tyr Gly Tyr
   485 490 495
   Thr Gly Tyr Pro Lys Ala Arg Trp Tyr Ser Lys Glu Cys Ala Glu Ser
   500 505 510
   Val Thr Ala Trp Gly Arg His Tyr Ile Glu Thr Thr Ile Asn Glu Ala
   515 520 525
   Glu Gly Phe Gly Phe Lys Val Leu Tyr Ala Asp Thr Asp Gly Phe Phe
   530 535 540
   Ala Thr Ile Pro Gly Glu Lys Pro Glu Val Ile Lys Lys Lys Ala Leu
   545 550 555 560
   Glu Phe Leu Lys His Ile Asn Lys Lys Leu Pro Gly Met Leu Glu Leu
   565 570 575
   Glu Tyr Glu Gly Phe Tyr Thr Arg Gly Phe Phe Val Thr Lys Lys Lys
   580 585 590
   Tyr Ala Leu Ile Asp Glu Glu Gly His Ile Thr Thr Arg Gly Leu Glu
   595 600 605
   Val Val Arg Arg Asp Trp Ser Glu Ile Ala Lys Glu Thr Xaa Ala Lys
   610 615 620
   Val Leu Glu Val Ile Leu Arg Glu Gly Ser Ile Glu Lys Ala Ala Gly
   625 630 635 640
   Ile Val Lys Lys Val Val Glu Asp Leu Ala Asn Tyr Arg Val Pro Val
   645 650 655
   Glu Lys Leu Val Ile His Glu Gln Ile Thr Arg Glu Leu Lys Asp Tyr
   660 665 670
   Lys Ala Thr Gly Pro His Val Ala Ile Ala Lys Arg Leu Gln Ala Arg
   675 680 685
   Gly Ile Lys Val Lys Pro Gly Thr Ile Ile Ser Tyr Val Val Leu Lys
   690 695 700
   Gly Ser Lys Lys Ile Ser Asp Arg Val Ile Leu Phe Asp Glu Tyr Asp
   705 710 715 720
   Pro Gly Arg His Lys Tyr Asp Pro Asp Tyr Tyr Ile His Asn Gln Val
   725 730 735
   Leu Pro Ala Val Leu Arg Ile Leu Glu Ala Phe Gly Tyr Lys Glu Lys
   740 745 750
   Asp Leu Glu Tyr Gln Arg Met Arg Gln Met Gly Leu Gly Ala Trp Leu
   755 760 765
   Gly Thr Gly Lys Gly
   770
<210> 2
   <211> 2573
   <212> DNA
   <213> Palaeococcus helgesonii
<220>
   <221> misc_feature
   <222> (1865)..(1865)
   <223> n is a, c, g, or t
<400> 2
   atgatacttg atacagatta tataacggag aatggaaaac ccgttatcag gatttttaag 60
   aaggaaaacg gcgagtttaa aatagaatac gacaggaatt ttgagcccta catttacgcg 120
   cttctggaga atgaggagga aatagaggac attaaaagga taaccgccga gaggcacgga 180
   aaaaaagtga gaatcgtgcg ggctgagaag gttaagaaaa agttcctggg agagcccata 240
   gaggtgtgga agcttgtttt tgagcatcca caggacgtcc cggacattat aaggaagcat 300
   cctgccgttg tggacatcta cgagtacgat atacccttcg caaagcgcta cctcatagac 360
   agagggcttg ttccgatgga gggcgacgag gagctcaaaa tgctggcttt tgatattgag 420
   acgttctacc atgagggaga tgaattcgga gagggcgaaa ttttgatgat aagctacgcc 480
   gatgagggcg gcgcgagggt gattacgtgg aagagaattg acctccccta tgtggaaacg 540
   gtatccacag agagggaagc cataaagcgc ttcctccatg ttctgaagga aaaagatccg 600
   gacgtgctca tcacgtacaa cggcgacaac ttcgattttg cttacataaa aaagcgctgt 660
   gaaaagctcg ggttgaagtt cacaatcggg agggacggaa gcgaaccaaa aattcagagg 720
   atgggggatc gcttcgccgt cgaggtcaag ggcatcaagg gcagaataca ccttgatctc 780
   tatcccgtcg tgaggcacac aataaggctc cccacctata cgcttgaggc ggtctatgaa 840
   gccgttttcg gaaagcgaaa ggagaaggtc tatgcagaag agatagcgac ggcatggaag 900
   agtgaggagg ggcttaagag ggtcgcgcag tattcaatgg aggatgcaaa agccacatat 960
   gagctcggaa gggagttctt cccgatggag gtggaactgg caaagctcat agggcagagc 1020
   gtttgggacg tatcgaggtc aagcacgggc aacctggtgg agtggtacct cctgagagag 1080
   gcatatgaga ggaacgagct cgcaccgaat aagccggggg atgcggaata caggaaaaga 1140
   atgcgctctt cctatctcgg gggctacgtc aaggagcccg agaaaggatt atgggagagc 1200
   atagcttatt tagattttcg cagcttgtac ccctccataa tcgtcaccca caacgtttct 1260
   cccgatacgc ttgaaagaga atgcaaaaac tattatgtgg ctccagttgt tggctaccgc 1320
   ttctgcagtg actttaaggg attcatccca agcatcctgg aggagctcat agaaaccagg 1380
   cagaaggtta agaggaagat gaaggccacg attgaccccg tggagaggaa gatgctcgac 1440
   tacaggcaga gggcattgaa gattctggcg aatagctatt acggttatac gggctatcca 1500
   aaagcgcgct ggtattcgaa ggagtgtgcc gagagcgtca cggcatgggg gaggcactac 1560
   atagagacca ctatcaatga ggcagaggga ttcgggttta aagtgctcta tgcggacact 1620
   gatggctttt ttgcaacaat acccggtgaa aaaccggagg tcataaaaaa gaaggccttg 1680
   gaattcctga aacacataaa taaaaagctc cccggaatgc tcgagcttga gtatgagggc 1740
   ttctacacga ggggattctt cgtcaccaaa aagaagtacg ctctcattga tgaggagggg 1800
   cacataacca cgaggggcct tgaggttgtg aggagggact ggagtgagat agcaaaggaa 1860
   acccnagcta aagtgctgga ggtcatctta agggagggta gcattgaaaa ggcagcgggg 1920
   atcgtgaaga aagttgttga ggatctggca aattaccgcg ttcccgtaga aaagctggtc 1980
   attcacgagc agattacccg ggaattaaag gattataagg cgacgggacc ccacgtggcg 2040
   atagcaaagc gccttcaggc aaggggcatc aaggtgaagc ccggcaccat aataagctat 2100
   gttgttttga aggggagcaa gaagataagc gacagggtaa tcctgttcga tgagtacgac 2160
   cccggcaggc ataagtatga cccagattac tacatccaca atcaggttct ccccgcggtt 2220
   cttagaatac tcgaagcctt cggatacaag gagaaagatc tggagtacca gaggatgaga 2280
   cagatgggac ttggggcgtg gcttggaacg gggaaggggt gagaggaaat atgccggtaa 2340
   aagcctcatg gaattacttc ttccatcctt tcgtagattc cggcttttct caaaacctca 2400
   cggcatgggg gaggcactac atagagacca ctatcaatga ggcagaggga ttcgggttta 2460
   aagtgctcta tgcggacact gatggctttt ttgcaacaat acccggtgaa aaaccggagg 2520
   tcataaaaaa gaaggccttg gaattcctga aacacataaa taaaaagctc ccc 2573
<210> 3
   <211> 29
   <212> DNA
   <213> Artificial
<220>
   <223> Primer sequence
<220>
   <221> misc_feature
   <222> (18)..(18)
   <223> n is a, c, g, or t
<400> 3
   cgcgggagaa cctggttntc datrtarta 29
<210> 4
   <211> 9
   <212> PRT
   <213> Unknown
<220>
   <223> Protein fragment
<400> 4
   Tyr Tyr Ile Glu Asn Gln Val Leu Pro
   1 5
<210> 5
   <211> 29
   <212> DNA
   <213> Artificial
<220>
   <223> Primer sequence
<220>
   <221> misc_feature
   <222> (21)..(21)
   <223> n is a, c, g, or t
<400> 5
   tactacggat aggccaargc nagrtggta 29
<210> 6
   <211> 9
   <212> PRT
   <213> Unknown
<220>
   <223> Protein fragment
<220>
   <221> MISC_FEATURE
   <222> (4)..(4)
   <223> Xaa is STOP
<400> 6
   Tyr Tyr Gly Xaa Ala Lys Ala Arg Trp
   1 5
<210> 7
   <211> 18
   <212> DNA
   <213> Artificial
<220>
   <223> Primer sequence
<400> 7
   tgtaaaacga cggccagt 18
<210> 8
   <211> 24
   <212> DNA
   <213> Artificial
<220>
   <223> Primer sequence
<400> 8
   agcggataac aatttcacac agga 24
<210> 9
   <211> 17
   <212> DNA
   <213> Artificial
<220>
   <223> Primer sequence
<400> 9
   catccacagg acgtccc 17
<210> 10
   <211> 6
   <212> PRT
   <213> Unknown
<220>
   <223> Protein fragment
<400> 10
   His Pro Gln Asp Val Pro
   1               5
<210> 11
   <211> 20
   <212> DNA
   <213> Artificial
<220>
   <223> Primer sequence
<400> 11
   taaacccgaa tccctctgcc 20
<210> 12
   <211> 20
   <212> DNA
   <213> Artificial
<220>
   <223> Primer sequence
<400> 12
   ttgtgtgcct cacgacggga 20
<210> 13
   <211> 22
   <212> DNA
   <213> Artificial
<220>
   <223> Primer sequence
<400> 13
   gcaaggggca tcaaggtgaa gc 22
<210> 14
   <211> 22
   <212> DNA
   <213> Artificial
<220>
   <223> Primer sequence
<400> 14
   tgttttgaag gggagcaaga ag 22
<210> 15
   <211> 22
   <212> DNA
   <213> Artificial
<220>
   <223> Primer sequence
<400> 15
   gcttttctac gggaacgcgg ta 22
<210> 16
   <211> 20
   <212> DNA
   <213> Artificial
<220>
   <223> Primer sequence
<400> 16
   gtgacgctct cggcacactc 20
<210> 17
   <211> 20
   <212> DNA
   <213> Artificial
<220>
   <223> Primer sequence
<400> 17
   ccacaacggc aggatgcttc 20
<210> 18
   <211> 20
   <212> DNA
   <213> Artificial
<220>
   <223> Primer sequence
<400> 18
   tagatgtcca caacggcagg 20
<210> 19
   <211> 20
   <212> DNA
   <213> Artificial
<220>
   <223> Primer sequence
<400> 19
   cagagggctt gttccgatgg 20
<210> 20
   <211> 40
   <212> DNA
   <213> Artificial
<220>
   <223> Primer sequence
<400> 20
   aagcttccat gggtattctt gatacagatt atataacgga 40
<210> 21
   <211> 39
   <212> DNA
   <213> Artificial
<220>
   <223> Primer sequence
<400> 21
   ggatccgtcg acttacccct tccccgttcc aagccacgc 39
<210> 22
   <211> 55
   <212> DNA
   <213> Artificial
<220>
   <223> Primer sequence
<400> 22
   ttcccctcta gaaataattt tgtttaactt taagaaggag atatacatat gcacc 55
<210> 23
   <211> 55
   <212> DNA
   <213> Artificial
<220>
   <223> Primer sequence
<400> 23
   gaattcggat ccgctagcca tggtatggtg atggtgatgg tgcatatgta tatct 55
<210> 24
   <211> 24
   <212> DNA
   <213> Artificial
<220>
   <223> Primer sequence
<400> 24
   aaattaatac gactcactat aggg 24
<210> 25
   <211> 19
   <212> DNA
   <213> Artificial
<220>
   <223> Primer sequence
<400> 25
   gctagttatt gctcagcgg 19
<210> 26
   <211> 20
   <212> DNA
   <213> Artificial
<220>
   <223> Primer sequence
<400> 26
   cctgctctgc cgcttcacgc 20
<210> 27
   <211> 24
   <212> DNA
   <213> Artificial
<220>
   <223> Primer sequence
<400> 27
   ccatgattca gtgtgcccgt ctgg 24
<210> 28
   <211> 23
   <212> PRT
   <213> Artificial
<220>
   <223> consensus sequence
<220>
   <221> MISC_FEATURE
   <222> (2)..(2)
   <223> Xaa is Lys or Arg
<220>
   <221> MISC_FEATURE
   <222> (3)..(3)
   <223> Xaa is Glu or Asp
<220>
   <221> MISC_FEATURE
   <222> (4)..(4)
   <223> Xaa is Met or Ala
<220>
   <221> MISC_FEATURE
   <222> (7)..(7)
   <223> Xaa is Arg or Ala
<220>
   <221> MISC_FEATURE
   <222> (10)..(10)
   <223> Xaa is Lys, Arg or His
<220>
   <221> MISC_FEATURE
   <222> (11)..(11)
   <223> Xaa is Val or Ile
<220>
   <221> MISC_FEATURE
   <222> (12)..(12)
   <223> Xaa is Val, Ile or Leu
<220>
   <221> MISC_FEATURE
   <222> (13)..(13)
   <223> Xaa is Lys or Arg
<220>
   <221> MISC_FEATURE
   <222> (19)..(19)
   <223> Xaa is Val or Ile
<220>
   <221> MISC_FEATURE
   <222> (20)..(20)
   <223> Xaa is Leu or Ile
<220>
   <221> MISC_FEATURE
   <222> (21)..(21)
   <223> Xaa is Val or Ile
<400> 28
   Glu Xaa Xaa Xaa Ile Lys Xaa Phe Leu Xaa Xaa Xaa Xaa Glu Lys Asp
   1               5                   10                  15
   Pro Asp Xaa Xaa Xaa Thr Tyr
               20
<210> 29
   <211> 36
   <212> PRT
   <213> Artificial
<220>
   <223> Consensus sequence
<220>
   <221> MISC_FEATURE
   <222> (2)..(2)
   <223> Xaa is Tyr or Phe
<220>
   <221> MISC_FEATURE
   <222> (8)..(8)
   <223> Xaa is Lys or Arg
<220>
   <221> MISC_FEATURE
   <222> (12)..(12)
   <223> Xaa is Glu or Asp
<220>
   <221> MISC_FEATURE
   <222> (13)..(13)
   <223> Xaa is Asn, Gly or Ser
<220>
   <221> MISC_FEATURE
   <222> (14)..(14)
   <223> Xaa is Leu or Ile
<220>
   <221> MISC_FEATURE
   <222> (15)..(15)
   <223> Xaa is Val or Ala
<220>
   <221> MISC_FEATURE
   <222> (16)..(16)
   <223> Xaa is Tyr or Ser
<220>
   <221> MISC_FEATURE
   <222> (19)..(19)
   <223> Xaa is Tyr or Phe
<220>
   <221> MISC_FEATURE
   <222> (20)..(20)
   <223> Xaa is Lys or Arg
<220>
   <221> MISC_FEATURE
   <222> (21)..(21)
   <223> Xaa is Ser or Ala
<220>
   <221> MISC_FEATURE
   <222> (28)..(28)
   <223> Xaa is Val or Ile
<400> 29
   Gly Xaa Val Lys Glu Pro Glu Xaa Gly Leu Trp Xaa Xaa Xaa Xaa Xaa
   1               5                   10                  15
   Leu Asp Xaa Xaa Xaa Leu Tyr Pro Ser Ile Ile Xaa Thr His Asn Val
               20                  25                  30
   Ser Pro Asp Thr
           35
<210> 30
   <211> 22
   <212> PRT
   <213> Artificial
<220>
   <223> Consensus sequence
<220>
   <221> MISC_FEATURE
   <222> (6)..(6)
   <223> Xaa is Leu or Ile
<220>
   <221> MISC_FEATURE
   <222> (8)..(8)
   <223> Xaa is Gly, Lys or Glu
<220>
   <221> MISC_FEATURE
   <222> (9)..(9)
   <223> Xaa is Asn, Asp, His or Glu
<220>
   <221> MISC_FEATURE
   <222> (11)..(11)
   <223> Xaa is Leu or Ile
<220>
   <221> MISC_FEATURE
   <222> (12)..(12)
   <223> Xaa is Glu or Asp
<220>
   <221> MISC_FEATURE
   <222> (13)..(13)
   <223> Xaa is Glu or Thr
<220>
   <221> MISC_FEATURE
   <222> (16)..(16)
   <223> Xaa is Lys or Glu
<220>
   <221> MISC_FEATURE
   <222> (17)..(17)
   <223> Xaa is Val or Ile
<220>
   <221> MISC_FEATURE
   <222> (19)..(19)
   <223> Xaa is Arg, Lys or Thr
<400> 30
   Gly Phe Ile Pro Ser Xaa Leu Xaa Xaa Leu Xaa Xaa Xaa Arg Gln Xaa
   1               5                   10                  15
   Xaa Lys Xaa Lys Met Lys
               20
<210> 31
   <211> 22
   <212> PRT
   <213> Artificial
<220>
   <223> Consensus sequence
<220>
   <221> MISC_FEATURE
   <222> (5)..(5)
   <223> Xaa is Lys or Arg
<220>
   <221> MISC_FEATURE
   <222> (7)..(7)
   <223> Xaa is Leu or Ile
<220>
   <221> MISC_FEATURE
   <222> (9)..(9)
   <223> Xaa is Leu or Ile
<220>
   <221> MISC_FEATURE
   <222> (14)..(14)
   <223> Xaa is Tyr or Phe
<220>
   <221> MISC_FEATURE
   <222> (18)..(18)
   <223> Xaa is Tyr or Thr
<220>
   <221> MISC_FEATURE
   <222> (21)..(21)
   <223> Xaa is Ala or Pro
<220>
   <221> MISC_FEATURE
   <222> (22)..(22)
   <223> Xaa is Lys or Arg
<400> 31
   Asp Tyr Arg Gln Xaa Ala Xaa Lys Xaa Leu Ala Asn Ser Xaa Tyr Gly
   1               5                   10                  15
   Tyr Xaa Gly Tyr Xaa Xaa
               20
<210> 32
   <211> 7
   <212> PRT
   <213> Artificial
<220>
   <223> Consensus sequence
<220>
   <221> MISC_FEATURE
   <222> (5)..(5)
   <223> Xaa is Phe or Leu
<220>
   <221> MISC_FEATURE
   <222> (6)..(6)
   <223> Xaa is Tyr, Phe or His
<400> 32
   Asp Thr Asp Gly Xaa Xaa Ala
   1               5
<210> 33
   <211> 23
   <212> PRT
   <213> Artificial
<220>
   <223> Consensus sequence
<220>
   <221> MISC_FEATURE
   <222> (5)..(5)
   <223> Xaa is Gly or His
<220>
   <221> MISC_FEATURE
   <222> (6)..(6)
   <223> Xaa is Val or Ile
<220>
   <221> MISC_FEATURE
   <222> (7)..(7)
   <223> Xaa is Val, Thr or Ile
<220>
   <221> MISC_FEATURE
   <222> (13)..(13)
   <223> Xaa is Val or Ile
<220>
   <221> MISC_FEATURE
   <222> (20)..(20)
   <223> Xaa is Glu or Asp
<400> 33
   Asp Glu Glu Gly Xaa Xaa Xaa Thr Arg Gly Leu Glu Xaa Val Arg Arg
   1               5                   10                  15
   Asp Trp Ser Xaa Ile Ala Lys
               20
<210> 34
   <211> 15
   <212> PRT
   <213> Artificial
<220>
   <223> Consensus sequence
<220>
   <221> MISC_FEATURE
   <222> (7)..(7)
   <223> Xaa is Val or Ile
<400> 34
   Leu Tyr Pro Ser Ile Ile Xaa Thr His Asn Val Ser Pro Asp Thr
   1               5                   10                  15
<210> 35
   <211> 16
   <212> PRT
   <213> Artificial
<220>
   <223> Consensus sequence
<220>
   <221> MISC_FEATURE
   <222> (6)..(6)
   <223> Xaa is Val or Ile
<220>
   <221> MISC_FEATURE
   <222> (13)..(13)
   <223> Xaa is Glu or Asp
<400> 35
   Thr Arg Gly Leu Glu Xaa Val Arg Arg Asp Trp Ser Xaa Ile Ala Lys
   1               5                   10                  15
<210> 36
   <211> 2322
   <212> DNA
   <213> Palaeococcus helgesonii
<400> 36
   atgatacttg atacagatta tataacggag aatggaaaac ccgttatcag gatttttaag      60
   aaggaaaacg gcgagtttaa aatagaatac gacaggaatt ttgagcccta catttacgcg     120
   cttctggaga atgaggagga aatagaggac attaaaagga taaccgccga gaggcacgga     180
   aaaaaagtga gaatcgtgcg ggctgagaag gttaagaaaa agttcctggg agagcccata     240
   gaggtgtgga agcttgtttt tgagcatcca caggacgtcc cggacattat aaggaagcat     300
   cctgccgttg tggacatcta cgagtacgat atacccttcg caaagcgcta cctcatagac      360
   agagggcttg ttccgatgga gggcgacgag gagctcaaaa tgctggcttt tgatattgag     420
   acgttctacc atgagggaga tgaattcgga gagggcgaaa ttttgatgat aagctacgcc     480
   gatgagggcg gcgcgagggt gattacgtgg aagagaattg acctccccta tgtggaaacg      540
   gtatccacag agagggaagc cataaagcgc ttcctccatg ttctgaagga aaaagatccg     600
   gacgtgctca tcacgtacaa cggcgacaac ttcgattttg cttacataaa aaagcgctgt     660
   gaaaagctcg ggttgaagtt cacaatcggg agggacggaa gcgaaccaaa aattcagagg     720
   atgggggatc gcttcgccgt cgaggtcaag ggcatcaagg gcagaataca ccttgatctc     780
   tatcccgtcg tgaggcacac aataaggctc cccacctata cgcttgaggc ggtctatgaa     840
   gccgttttcg gaaagcgaaa ggagaaggtc tatgcagaag agatagcgac ggcatggaag     900
   agtgaggagg ggcttaagag ggtcgcgcag tattcaatgg aggatgcaaa agccacatat     960
   gagctcggaa gggagttctt cccgatggag gtggaactgg caaagctcat agggcagagc    1020
   gtttgggacg tatcgaggtc aagcacgggc aacctggtgg agtggtacct cctgagagag    1080
   gcatatgaga ggaacgagct cgcaccgaat aagccggggg atgcggaata caggaaaaga    1140
   atgcgctctt cctatctcgg gggctacgtc aaggagcccg agaaaggatt atgggagagc    1200
   atagcttatt tagattttcg cagcttgtac ccctccataa tcgtcaccca caacgtttct    1260
   cccgatacgc ttgaaagaga atgcaaaaac tattatgtgg ctccagttgt tggctaccgc    1320
   ttctgcagtg actttaaggg attcatccca agcatcctgg aggagctcat agaaaccagg    1380
   cagaaggtta agaggaagat gaaggccacg attgaccccg tggagaggaa gatgctcgac    1440
   tacaggcaga gggcattgaa gattctggcg aatagctatt acggttatac gggctatcca    1500
   aaagcgcgct ggtattcgaa ggagtgtgcc gagagcgtca cggcatgggg gaggcactac    1560
   atagagacca ctatcaatga ggcagaggga ttcgggttta aagtgctcta tgcggacact    1620
   gatggctttt ttgcaacaat acccggtgaa aaaccggagg tcataaaaaa gaaggccttg    1680
   gaattcctga aacacataaa taaaaagctc cccggaatgc tcgagcttga gtatgagggc    1740
   ttctacacga ggggattctt cgtcaccaaa aagaagtacg ctctcattga tgaggagggg    1800
   cacataacca cgaggggcct tgaggttgtg aggagggact ggagtgagat agcaaaggaa 1860
   acccaagcta aagtgctgga ggtcatctta agggagggta gcattgaaaa ggcagcgggg 1920
   atcgtgaaga aagttgttga ggatctggca aattaccgcg ttcccgtaga aaagctggtc 1980
   attcacgagc agattacccg ggaattaaag gattataagg cgacgggacc ccacgtggcg 2040
   atagcaaagc gccttcaggc aaggggcatc aaggtgaagc ccggcaccat aataagctat 2100
   gttgttttga aggggagcaa gaagataagc gacagggtaa tcctgttcga tgagtacgac 2160
   cccggcaggc ataagtatga cccagattac tacatccaca atcaggttct ccccgcggtt 2220
   cttagaatac tcgaagcctt cggatacaag gagaaagatc tggagtacca gaggatgaga 2280
   cagatgggac ttggggcgtg gcttggaacg gggaaggggt ga 2322
<210> 37
   <211> 81
   <212> DNA
   <213> Artificial
<220>
   <223> Plasmid fragment
<400> 37
   tctagaaata attttgttta actttaagaa ggagatatac atatgcacca tcaccatcac 60
   cataccatgg ctagcggatc c 81
<210> 38
   <211> 9
   <212> PRT
   <213> Artificial
<220>
   <223> Protein tag sequence
<400> 38
   Met His His His His His His Thr Met
   1 5
<210> 39
   <211> 773
   <212> PRT
   <213> Palaeococcus helgesonii
<400> 39
   Met Ile Leu Asp Thr Asp Tyr Ile Thr Glu Asn Gly Lys Pro Val Ile
   1               5                   10                  15
   Arg Ile Phe Lys Lys Glu Asn Gly Glu Phe Lys Ile Glu Tyr Asp Arg
               20                  25                  30
   Asn Phe Glu Pro Tyr Ile Tyr Ala Leu Leu Glu Asn Glu Glu Glu Ile
           35                  40                  45
   Glu Asp Ile Lys Arg Ile Thr Ala Glu Arg His Gly Lys Lys Val Arg
       50                  55                  60
   Ile Val Arg Ala Glu Lys Val Lys Lys Lys Phe Leu Gly Glu Pro Ile
   65                  70                  75                  80
   Glu Val Trp Lys Leu Val Phe Glu His Pro Gln Asp Val Pro Asp Ile
                   85                  90                  95
   Ile Arg Lys His Pro Ala Val Val Asp Ile Tyr Glu Tyr Asp Ile Pro
               100                 105                 110
   Phe Ala Lys Arg Tyr Leu Ile Asp Arg Gly Leu Val Pro Met Glu Gly
           115                 120                 125
   Asp Glu Glu Leu Lys Met Leu Ala Phe Asp Ile Glu Thr Phe Tyr His
       130                 135                 140
   Glu Gly Asp Glu Phe Gly Glu Gly Glu Ile Leu Met Ile Ser Tyr Ala
   145                 150                 155                 160
   Asp Glu Gly Gly Ala Arg Val Ile Thr Trp Lys Arg Ile Asp Leu Pro
                   165                 170                 175
   Tyr Val Glu Thr Val Ser Thr Glu Arg Glu Ala Ile Lys Arg Phe Leu
               180                 185                 190
   His Val Leu Lys Glu Lys Asp Pro Asp Val Leu Ile Thr Tyr Asn Gly
           195                 200                 205
   Asp Asn Phe Asp Phe Ala Tyr Ile Lys Lys Arg Cys Glu Lys Leu Gly
       210                 215                 220
   Leu Lys Phe Thr Ile Gly Arg Asp Gly Ser Glu Pro Lys Ile Gln Arg
   225                 230                 235                 240
   Met Gly Asp Arg Phe Ala Val Glu Val Lys Gly Ile Lys Gly Arg Ile
                   245                 250                 255
   His Leu Asp Leu Tyr Pro Val Val Arg His Thr Ile Arg Leu Pro Thr
               260                 265                 270
   Tyr Thr Leu Glu Ala Val Tyr Glu Ala Val Phe Gly Lys Arg Lys Glu
           275                 280                 285
   Lys Val Tyr Ala Glu Glu Ile Ala Thr Ala Trp Lys Ser Glu Glu Gly
       290                 295                 300
   Leu Lys Arg Val Ala Gln Tyr Ser Met Glu Asp Ala Lys Ala Thr Tyr
   305                 310                 315                 320
   Glu Leu Gly Arg Glu Phe Phe Pro Met Glu Val Glu Leu Ala Lys Leu
                   325                 330                 335
   Ile Gly Gln Ser Val Trp Asp Val Ser Arg Ser Ser Thr Gly Asn Leu
   340 345 350
   Val Glu Trp Tyr Leu Leu Arg Glu Ala Tyr Glu Arg Asn Glu Leu Ala
   355 360 365
   Pro Asn Lys Pro Gly Asp Ala Glu Tyr Arg Lys Arg Met Arg Ser Ser
   370 375 380
   Tyr Leu Gly Gly Tyr Val Lys Glu Pro Glu Lys Gly Leu Trp Glu Ser
   385 390 395 400
   Ile Ala Tyr Leu Asp Phe Arg Ser Leu Tyr Pro Ser Ile Ile Val Thr
   405 410 415
   His Asn Val Ser Pro Asp Thr Leu Glu Arg Glu Cys Lys Asn Tyr Tyr
   420 425 430
   Val Ala Pro Val Val Gly Tyr Arg Phe Cys Ser Asp Phe Lys Gly Phe
   435 440 445
   Ile Pro Ser Ile Leu Glu Glu Leu Ile Glu Thr Arg Gln Lys Val Lys
   450 455 460
   Arg Lys Met Lys Ala Thr Ile Asp Pro Val Glu Arg Lys Met Leu Asp
   465 470 475 480
   Tyr Arg Gln Arg Ala Leu Lys Ile Leu Ala Asn Ser Tyr Tyr Gly Tyr
   485 490 495
   Thr Gly Tyr Pro Lys Ala Arg Trp Tyr Ser Lys Glu Cys Ala Glu Ser
   500 505 510
   Val Thr Ala Trp Gly Arg His Tyr Ile Glu Thr Thr Ile Asn Glu Ala
   515 520 525
   Glu Gly Phe Gly Phe Lys Val Leu Tyr Ala Asp Thr Asp Gly Phe Phe
   530 535 540
   Ala Thr Ile Pro Gly Glu Lys Pro Glu Val Ile Lys Lys Lys Ala Leu
   545 550 555 560
   Glu Phe Leu Lys His Ile Asn Lys Lys Leu Pro Gly Met Leu Glu Leu
   565 570 575
   Glu Tyr Glu Gly Phe Tyr Thr Arg Gly Phe Phe Val Thr Lys Lys Lys
   580 585 590
   Tyr Ala Leu Ile Asp Glu Glu Gly His Ile Thr Thr Arg Gly Leu Glu
   595 600 605
   Val Val Arg Arg Asp Trp Ser Glu Ile Ala Lys Glu Thr Gln Ala Lys
       610                 615                 620
   Val Leu Glu Val Ile Leu Arg Glu Gly Ser Ile Glu Lys Ala Ala Gly
   625                 630                 635                 640
   Ile Val Lys Lys Val Val Glu Asp Leu Ala Asn Tyr Arg Val Pro Val
                   645                 650                 655
   Glu Lys Leu Val Ile His Glu Gln Ile Thr Arg Glu Leu Lys Asp Tyr
               660                 665                 670
   Lys Ala Thr Gly Pro His Val Ala Ile Ala Lys Arg Leu Gln Ala Arg
           675                 680                 685
   Gly Ile Lys Val Lys Pro Gly Thr Ile Ile Ser Tyr Val Val Leu Lys
       690                 695                 700
   Gly Ser Lys Lys Ile Ser Asp Arg Val Ile Leu Phe Asp Glu Tyr Asp
   705                 710                 715                 720
   Pro Gly Arg His Lys Tyr Asp Pro Asp Tyr Tyr Ile His Asn Gln Val
                   725                 730                 735
   Leu Pro Ala Val Leu Arg Ile Leu Glu Ala Phe Gly Tyr Lys Glu Lys
               740                 745                 750
   Asp Leu Glu Tyr Gln Arg Met Arg Gln Met Gly Leu Gly Ala Trp Leu
           755                 760                 765
   Gly Thr Gly Lys Gly
       770